# EUROPÄISCHE PATENTSCHRIFT

(11) **EP 0 866 123 B1**
(45) Veröffentlichungstag und Bekanntmachung des Hinweises auf die Patenterteilung: **16.03.2005**
(21) Anmeldenummer: 98103960.5
(22) Anmeldetag: 06.03.1998
(51) Int. Cl.: C12N 13/00, C12N 15/87, C12M 3/00

(54) **Verfahren und Schaltungsanordnung zur Elektropermeation oder Elektroporation von lebenden Zellen**
Method and circuit device for electropermeation or electroporation of living cells
Procédé et dispositif de circuit pour l'electropermeation ou l'electroporation de cellules vivantes

(30) Priorität: 21.03.1997 DE 19711854
(43) Veröffentlichungstag der Anmeldung: 23.09.1998
(73) Patentinhaber: EPPENDORF AG, 22339 Hamburg (DE)
(72) Erfinder: Kröger, Wolfgang, Dipl.-Ing., 23899 Gudow (DE); Jagdhuber, Bernd, 25436 Uetersen (DE); Ricklefs, Hans-Joachim, Dipl.-Ing., 22359 Hamburg (DE)
(74) Vertreter: Patentanwälte Hauck, Graalfs, Wehnert, Döring, Siemons, Schildberg

(56) Entgegenhaltungen:
- WO-A-87/06851
- DE-A- 3 718 941
- US-A- 5 098 843
- US-A- 5 273 525
- US-A- 5 439 440

## Beschreibung

Die Erfindung bezieht sich auf ein Verfahren zur Elektropermeation von lebenden Zellen nach dem Oberbegriff des Patentanspruchs 1.

Es ist bekannt, zum Zwecke der Detektion, Expression, Markierung, Strukturaufklärung, Anreicherung, Separation, Apotose, Genregulation, Gentherapie, Transformation, Zell-Zell-Wechselwirkung usw. Moleküle und Partikel in lebende Zellen einzubringen. Hierfür ist erforderlich, die Zellmembran vorübergehend durchlässig zu machen, ohne daß das Zellinnere und die Überlebensfähigkeit der Zelle merklich beeinträchtigt werden. Das derzeitige Verfahren der Wahl ist die Lipofektion. Ein anderes mögliches Verfahren ist die Elektroporation oder -permeation. Bei diesem Verfahren werden die lebenden Zellen in ein elektrisches Feld verhältnismäßig hoher Feldstärke, jedoch relativ kurzer Dauer gebracht. Bei richtiger Wahl der Parameter gelingt es, in die Zellmembran mindestens eine Mikroöffnung einzubringen, die durch Elektrophorese auf ein bestimmtes Maß erweitert wird. Über diese Öffnung ist dann möglich, Moleküle oder Partikel in die Zelle einzuschleusen. Als Moleküle kommen z.B. Nukleinsäuren, Proteine, Enzyme, Regulatorproteine, Liganden, Rezepturen, Antikörper, Kontrastmittel für Röntgen-, NMR- und Ultraschall-Untersuchungen und Elektronenmikroskopie in Frage. Partikel können z.B. sein Chromosomen, Viren, Organellen oder Zellen, magnetische Partikel, Gold, Kohlenstoff usw.

Die beschriebene Elektroporation findet sowohl in vitro als auch in vivo statt. Bei der in-vitro-Elektroporation befinden sich die lebenden Zellen in einer Suspension einer geeigneten Flüssigkeit, die in ein Probengefäß eingebracht ist (Küvette). Das Probengefäß ist mit Elektrodenplatten versehen (Plattenkondensator). Bei der in-vivo-Elektroporation werden Elektroden, beispielsweise in Form von Nadeln in Körperbereiche eingeführt. Die vorliegende Erfindung bezieht sich generell auf die Elektroporation.

Aus der GB 2 227 380 oder der DE 37 18 941 ist bekanntgeworden, an die Elektroden eines Probengefäßes eine Spannung in Form eines Rechteckimpulses vorgegebener Dauer und Höhe anzulegen. Der Strom ist größer als 100 Ampere, und die Spannung beträgt etwa 3000 Volt. Als Spannungsquelle dient ein Kondensator, der über Leistungsschalter auf die Elektroden des Probengefäßes geschaltet wird. Als Leistungsschalter dient eine Anzahl von parallelgeschalteten Thyristoren. Die Einschaltzeit der Thyristoren wird überwacht. Ist eine vorgegebene Zeit erreicht, werden die Thyristoren gesperrt.

Aus US 5 273 525 oder 5 439 440 ist eine in vivo-Elektroporation bekannt, bei der verschieden geformte Pulse an die Elektroden gelegt werden, beispielsweise Pulse mit exponentiell abfallender Flanke, Rechteckpulse, unipolare oder bipolare oszillierende Pulse, die zwischen den Elektroden eine Feldstärke von 0,2 kV/cm bis 20 kV/cm erzeugen. Die Pulslänge ist zwischen 10 und 100 ms, wobei bis zu 100 Pulse nacheinander erzeugt werden. In diesen Schriften wird auch erwähnt, daß die Parameter der Elektroporation danach auszuwählen sind, welche Bedingungen angetroffen werden, d.h. wie die Zellen beschaffen sind. Feldstärke und Dauer des Pulses müssen mithin variiert werden, um einerseits die gewünschte Permeation zu erzielen, andererseits eine Schädigung der Zellen zu vermeiden.

Es hat sich jedoch herausgestellt, daß es nicht ausreicht, den Verlauf der Spannung bzw. des Stroms an den Elektroden des Probengefäßes vorzugeben, indem ein Generator in entsprechender Weise angesteuert wird, weil die tatsächlichen Verhältnisse im Hinblick auf die verwendete Flüssigkeit und die Art und die Beschaffenheit der lebenden Zellen andere Feldstärken im Gefolge haben als durch die Spannungsvorgabe und Geometrie der Elektroden eintreten müßten. Die sich jeweils einstellende Feldstärke ist bekanntlich abhängig von den Dielektrizitätskonstanten der verschiedenen Medien im Probengefäß sowie von der Stromleitfähigkeit der Medien, welche Eigenschaften meist nicht exakt reproduzierbar herbeigeführt werden können.

Aus US-A-4,943,761 ist eine Schaltungsanordnung bekannt geworden mit einer Spannungs- und Stromquelle, die über einen als Stellelement arbeitenden steuerbaren Halbleiter an eine Last angeschlossen ist. Die Stellsignale des steuerbaren Halbleiters werden von einer Treiberstufe erzeugt, die mit einer elektronischen Regelschaltung verbunden ist. Eine Stromüberwachungsschaltung mißt den über die Last fließenden Strom, der mit einem vorgegebenen Stromwert verglichen wird zur Begrenzung des Stroms auf einen vorgegebenen Wert. Aus diesem Dokument ist ferner bekannt, die Schaltungsanordnung über einen Optokoppler in Betrieb zu setzen.

Der Erfindung liegt daher die Aufgabe zugrunde, ein Verfahren zur Elektropermeation von lebenden Zellen anzugeben, das wirksam arbeitet, jedoch zugleich die Überlebensrate der Zellen nachhaltig verbessert.

Diese Aufgabe wird durch die Merkmale des Patentanspruchs 1 gelöst.

Bei dem erfindungsgemäßen Verfahren wird der Verlauf des Potentials bzw. des Stroms über die Elektroden des Probengefäßes vorgegeben. Die optimalen Parameter für den Verlauf bzw. die Dauer des Intervalls werden empirisch ermittelt. Bei einer Ausgestaltung der Erfindung liegt das Intervall zwischen 15 und 500 µs, was relativ kurz ist gegenüber herkömmlichen Verfahren. Bei dem erfindungsgemäßen Verfahren wird ferner der Verlauf der Spannung bzw. des Stromes überwacht und mittels eines Istwertgebers einem Regler zugeführt, der die Signale für die Ansteuerung eines Leistungshalbleiters liefert, über den eine Spannungsquelle mit den Elektroden verbunden wird. Bei dem erfindungsgemäßen Verfahren wird mithin der Leistungshalbleiter nicht als Schalter verwendet, sondern als Stelloder Steuerglied zur Einstellung der an die Elektroden anzulegenden Spannung über das vorgegebene Zeitintervall.

Ergibt sich durch entsprechende Experimente, daß eine bestimmte Spannungsverlaufform bei wirksamer Elektroporation eine hohe Überlebensrate bei einer hohen Anzahl permeierter Zellen gewährleistet, dann ist das erfindungsgemäße Verfahren in der Lage, die korrespondierende elektrische Feldstärke bereitzustellen.

Eine Schaltungsanordnung für die Elektropermeation von lebenden Zellen sieht eine elektronische Regelschaltung vor, die Stellsignale für die Treiberstufe eines steuerbaren Halbleiters erzeugt. Nach einer Ausgestaltung der Erfindung ist dieser ein Insulated Gate Bipolar Transistor (IGBT), wie er aus der Leistungselektronik an sich bekannt ist, insbesondere bei Stromrichtern. Ein Istwertgeber erfaßt den Strom bzw. die Spannung an den Elektroden und liefert einen entsprechenden Istwert an die elektronische Regelschaltung. Vom Sollwertgeber, der einen beliebigen Kurvenverlauf erzeugen kann, wird die Verlaufsform der Spannung bzw. des Stromes über ein vorgegebenes Intervall vorgegeben.

Die Spannungs- bzw. Stromquelle kann von einem Kondensator gebildet sein, wie dies aus dem Stand der Technik an sich bekannt ist. Für die Erfindung wesentlich ist jedoch, daß die Kapazität des Kondensators ausreichend groß bemessen ist, so daß er im Hinblick auf das Zeitintervall als nicht erschöpfbare Quelle dient. Die Form des an die Elektroden zu legenden Pulses wird allein durch den Sollwertgeber bestimmt. Dieser wird von einem Rechner gebildet, dessen z.B. stufenförmiges Ausgangssignal über einen Digital-Analogwandler auf die elektronische Regelschaltung gegeben wird. Mit Hilfe des Ausgangssignals des Rechners läßt sich jede beliebige Kurve erzeugen, indem das z.B. gestufte Signal in ein Analogsignal umgewandelt wird. So kann beispielsweise der Puls einen exponentiell abfallenden Verlauf aufweisen, wie grundsätzlich an sich ebenfalls bekannt ist. Die Länge der Stufen des Ausgangssignals richtet sich danach, wie eine Annäherung an den gewünschten Kurvenverlauf am günstigsten erreichbar ist.

Es ist ferner an sich bekannt, die Steuerelektronik von der Treiberstufe für einen Halbleitersteller galvanisch zu trennen. Zu diesem Zweck kann ein Transformator oder ein Optokoppler verwendet werden. Nach einer Ausgestaltung der Erfindung sieht die Ankopplung der Regelschaltung an die Treiberstufe eine Parallelschaltung aus Optokoppler und Transformator vor, wobei die Ausgangssignale dieser Elemente in einer Summierstufe summiert werden. Mit Hilfe beider Koppelelemente wird der Tatsache Rechnung getragen, daß Pulse gewünschter Länge mit äußerst steiler Anstiegsflanke übertragen werden, was der Schnelligkeit der Regelung zugute kommt.

Die Erfindung wird nachfolgend anhand eines Ausführungsbeispiels näher erläutert.

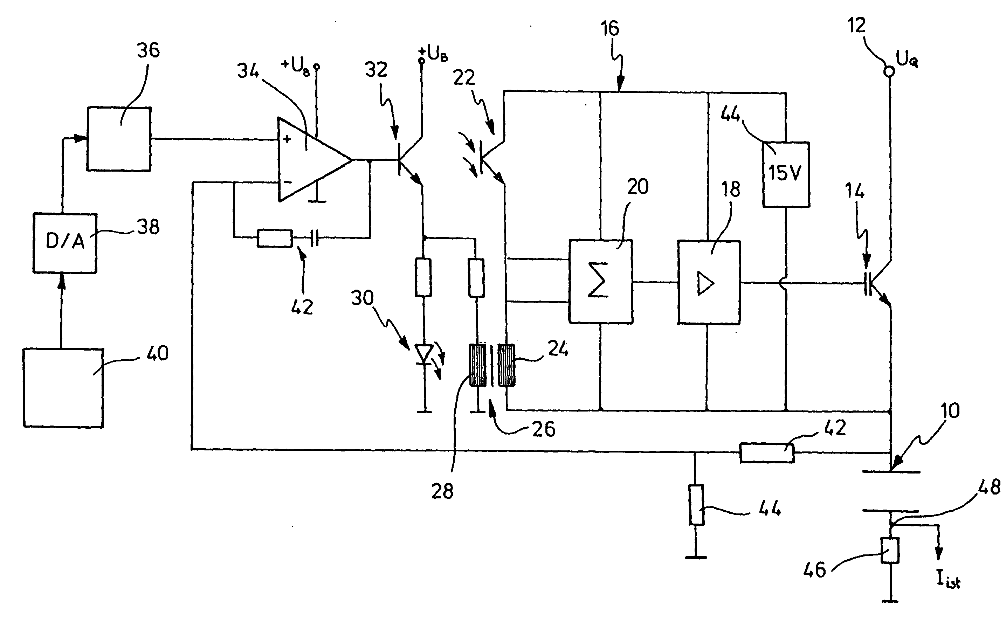

Die einzige Figur zeigt eine Schaltungsanordnung zur Elektropermeation von lebenden Zellen nach der Erfindung.

In der Zeichnung bezeichnet 10 die Elektroden an einem Probengefäß, die eine Art Plattenkondensator bilden mit vorgegebenem Abstand. Die Elektroden 10 werden an eine an einer Klemme 12 erzeugten Klemmenspannung U_{Q} gelegt, und zwar über einen IGBT 14. Die Spannungsquelle kann z.B. ein Kondensator relativ großer Kapazität sein (nicht gezeigt), der über einen nicht gezeigten Schalter an die Klemme 12 anlegbar ist.

Eine Treiberstufe 16 für den IGBT weist einen Verstärker 18 auf, dessen Eingang mit einer Summierstufe 20 verbunden ist, deren Eingänge einmal mit dem Emitter eines lichtempfindlichen Transistors 22 und zum anderen mit der Sekundärspule 24 eines Transformators 26 verbunden sind. Die Primärspule 28 des Transformators 26 liegt parallel mit einer lichtemittierenden Diode 30, die mit dem Transistor 22 einen Optokoppler bildet. Diode 30 und Primärspule 28 sind mit dem Emitter eines Transistors 32 verbunden, dessen Kollektor an Betriebsspannung U_{B} liegt. Die Basis des Transistors 32 ist mit dem Ausgang eines Operationsverstärkers 34 verbunden. Der positive Eingang des Operationsverstärkers 34 ist mit einem Sollwertgeber 36 verbunden, der über einen Digital-Analogwandler 38 an einen Rechner 40 angeschlossen ist. Der negative Eingang des Operationsverstärkers 34 ist über einen Widerstand 42 mit einem Punkt zwischen IGBT 14 und Elektroden 10 angeschlossen. Der Widerstand 42 bildet mit einem parallel zu den Elektroden 10 liegenden Widerstand 44 einen Spannungsteiler. Zwischen Elektroden 10 und Masse ist ein Shunt 46 geschaltet zwecks Erfassung des über die Elektroden 10 fließenden Stroms.

Es sei angenommen, daß die Elektroden 10 mit einer Spannung versorgt werden soll, die etwa in einem Intervall von 15 bis 500 µs anliegt und einen exponentiell abfallenden Verlauf hat. Zu diesem Zweck muß der Schalter, der die nicht gezeigte Spannungsquelle mit der Klemme 12 verbindet, geschlossen werden. Dieser kann über den Rechner 40 gesteuert werden. Gleichzeitig erzeugt der Rechner 40 die Sollfunktion durch Vorgabe einer stufenartig verlaufenden Kurve derart, daß sie der gewünschten Kurve weitgehend angenähert ist, wenn sie über den Digital-Analogwandler 38 in ein Analogsignal umgewandelt ist. Über den Analog-Sollwertgeber 36 wird dieses Signal auf den Operationsverstärker 34 gegeben, dieser erzeugt mit Hilfe des Transistors 32 und der beschriebenen Ankopplung an die Treiberstufe 16 ein Stellsignal für die Treiberstufe 16, die den IGBT nach Maßgabe des Sollsignals so ansteuert, daß die an den Elektroden 10 abfallende Spannung den gewünschten Verlauf und die gewünschte Größe aufweist. Da dies, wie eingangs erwähnt, nicht automatisch der Fall ist, wird die Spannung über den Spannungsteiler 42, 44 auf den Eingang des Operationsverstärkers 34 rückgekoppelt, um eine Regelung herbeizuführen.

Es sei noch erwähnt, daß der Verstärkungsfaktor durch den Rückkopplungskreis 42 eingestellt ist. Der IGBT 14 kann aus einer Mehrzahl von parallelgeschalteten IGBTs bestehen. Die Stufe 44 sorgt für einen Spannungsoffset.

Um Schäden zu vermeiden, ist es zweckmäßig, den Strom zu überwachen, was durch Abgriff eines Strommessers bei 48 der Fall ist. Überschreitet der Strom ein vorgegebenes Maß, wird die Spannungsquelle abgeschaltet.

Es ist auch denkbar, statt der beschriebenen Spannungsregelung eine Stromregelung durchzuführen. Zu diesem Zweck wird dann der Stromistwert auf die elektronische Regelung gegeben, die vom Prinzip her ähnlich aufgebaut ist wie für die Spannungsregelung.

## Patentansprüche

1. "in Vitro" Verfahren zur Elektropermeation von lebenden Zellen zwischen mindestens zwei Elektroden (10) über ein vorgegebenes Intervall an ein elektrisches Potential oder einen Strom, **gekennzeichnet durch** folgende Merkmale:
- Eine über das Intervall nicht erschöpfbare Spannungs- oder Stromquelle wird über einen als Steller arbeitenden steuerbaren Leistungshalbleiter (14) an die Elektroden gelegt;
- Der Verlauf des Potentials oder des Stroms während des Intervalls wird von einem Sollwertgeber, der einen beliebigen wählbaren Kurvenverlauf generiert, vorgegeben und auf einen der Treiberstufe (16) für den Leistungshalbleiter (14) vorgeschalteten Regler (34) gegeben;
- Der Regler (34) erhält ein dem tatsächlichen an den Elektroden (10) anliegenden Potential oder Strom entsprechenden Wert als Istwert;
- Das Intervall beträgt einen Zeitbereich von wenigen 100 ns bis mehrere Minuten.

2. Verfahren nach Anspruch 1, **dadurch gekennzeichnet, daß** bei Wiederholung der Abstand zwischen den Intervallen jeweils mindestens eine Sekunde beträgt.

3. Verfahren nach einem der Ansprüche 1 bis 3, **dadurch gekennzeichnet, daß** das Intervall zwischen 15 und 500 µs beträgt.

4. Schaltungsanordnung für die Elektropermeation von lebenden Zellen zwischen mindestens zwei Elektroden, mit einer Spannungs- oder Stromquelle, die während eines vorgegebenen Intervalls über einen steuerbaren Halbleiter mit den Elektroden verbindbar ist und einer Vorrichtung zur Ansteuerung des steuerbaren Halbleiters, **dadurch gekennzeichnet, daß** die Spannungs- oder Stromquelle über einen als Stellelement arbeitenden steuerbaren Halbleiter (14) mit den Elektroden (10) verbunden ist, die Stellsignale für die Treiberstufe (16) des steuerbaren Halbleiters (14) von einer elektronischen Regelschaltung erzeugt werden, mit der ein den Verlauf des Potentials oder des Stroms vorgebender Sollwertgeber (36) sowie ein das Potential oder den Strom an den Elektroden (10) erfassender Istwertgeber verbunden sind, wobei der Sollwertgeber einen Rechner (40) umfaßt, dessen Stufenförmiges Ausgangssignal über einen Digital-Analogwandler (38) auf die Regelschaltung gegeben wird und der Ausgang der elektronischen Regelschaltung über eine Potentialtrennstufe (22, 30; 26) mit der Treiberstufe (16) gekoppelt ist.

5. Schaltungsanordnung nach Anspruch 4, **dadurch gekennzeichnet, daß** der steuerbare Halbleiter (14) ein IGBT ist.

6. Schaltungsanordnung nach Anspruch 4 oder 5, **dadurch gekennzeichnet, daß** die Ankopplung der Regelschaltung an die Treiberstufe (16) parallel über einen Optokoppler (30, 22) und einen Transformator (26), vorzugsweise Pulstrans**formator, erfolgt und die Ausgangs**signale von Optokoppler und Transformator in einer Summierstufe (20) summiert werden.

## Claims

1. An "in-vitro" method for the electropermeation of living cells between at least two electrodes (10) to an electric potential or a current via a predetermined interval, **characterized by** the features which follow:
- A voltage or current source which is not exhaustible over the interval is applied to the electrodes via a controllable power semiconductor (14) operating as an actuator;
- The characteristic of the potential or current during the interval is predetermined by a setpoint generator which generates a random selectable curve shape, and is provided to a controller (34) connected ahead of the driver stage (16) for the power semiconductor (14);
- The controller (34) receives a value, which corresponds to the actual potential or current applied to the electrodes (10), as an instantaneous value.
- The interval has a time range of a few 100 ns to several minutes.

2. The method according to claim 2, **characterized in that** the distance between the intervals, if repeated, is at least one second each.

3. The method according to any one of claims 1 to 3, **characterized in that** the interval is between 15 and 500 µs.

4. A circuit arrangement for the electropermeation of living cells between at least two electrodes, with a voltage or current source which is adapted to be connected to the electrodes via a controllable semiconductor during a predetermined interval, and a device for triggering the controllable semiconductor, **characterized in that** the voltage or current source is connected to the electrodes (10) via a controllable power semiconductor (14) operating as an actuator, the setting signals for the driver stage (16) of the controllable power semiconductor (14) are produced by an electronic control circuit which has connected thereto a setpoint generator (36) predetermining the course of the potential or current and an actual-value transducer detecting the potential or current at the electrodes (10) wherein the setpoint generator comprises a computer (40) the stepped output signal of which is provided to the control circuit via a digital-to-analog converter (38) and the output of the electronic control circuit is coupled to the driver stage (16) via a potential separating stage (22, 30; 26).

5. The circuit arrangement according to claim 4, **characterized in that** the controllable semiconductor (14) is an IGBT.

6. The circuit arrangement according to claim 4 or 5, **characterized in that** the control circuit is coupled to the driver stage (16) in parallel via an opto-coupler (30, 32) and a transformer (26), preferably a pulse transformer, and the output signals of the opto-coupler and transformer are summed up at a summation stage (20).

## Revendications

1. Procédé " in vitro " pour l'électroperméation de cellules vivantes entre au moins deux électrodes (10) soumises à un potentiel électrique ou à un courant pendant un intervalle prédéterminé, **caractérisé par** les caractéristiques suivantes :
- une source de tension ou de courant inépuisable pendant l'intervalle est connectée aux électrodes par l'intermédiaire d'un semi-conducteur de puissance (14) contrôlable fonctionnant comme un moyen de réglage ;
- la variation du potentiel ou du courant pendant l'intervalle est spécifié par un ajusteur de valeur de consigne permettant de générer une courbe de variation quelconque, et fournie à un régulateur (34) placé en amont d'un étage d'attaque (16) pour le semi-conducteur de puissance (14) ;
- le régulateur (34) utilise en tant que valeur instantanée une valeur correspondant au potentiel ou au courant effectif aux électrodes (10);
- l'intervalle s'étend sur un domaine temporel de moins de 100 ns à plusieurs minutes.

2. Procédé selon la revendication 1, **caractérisé en ce que**, en cas de répétition, l'écart entre les intervalles dure à chaque fois au moins une seconde.

3. Procédé selon l'une des revendications 1 à 2, **caractérisé en ce que** l'intervalle dure entre 15 et 500 µs.

4. Circuit pour l'électroperméation de cellules vivantes entre au moins deux électrodes, comportant une source de tension ou de courant qui, pendant un intervalle prédéterminé, peut être raccordée par l'intermédiaire d'un semi-conducteur contrôlable aux électrodes, et un dispositif pour la commande du semi-conducteur contrôlable, **caractérisé en ce que** la source de tension ou de courant est raccordée aux électrodes (10) par l'intermédiaire d'un semi-conducteur (14) contrôlable fonctionnant comme un élément de réglage, les signaux de réglage pour l'étage d'attaque (16) du semi-conducteur contrôlable (14) sont générés par un circuit de réglage électronique, auquel sont raccordés un ajusteur de valeur de consigne (36) spécifiant la variation du potentiel ou du courant ainsi qu'un module fournissant des valeurs instantanées du potentiel ou du courant au niveau des électrodes (10), l'ajusteur de valeur de consigne comprenant un calculateur (40), dont le signal de sortie en gradins est donné, par l'intermédiaire d'un convertisseur numérique/analogique (38), au circuit de réglage et la sortie du circuit de réglage électronique est couplée, par l'intermédiaire d'un étage de séparation du potentiel (22, 30 ; 26), à l'étage d'attaque (16).

5. Circuit selon la revendication 4, **caractérisé en ce que** le semi-conducteur (14) contrôlable est un IGBT.

6. Circuit selon la revendication 4 ou 5, **caractérisé en ce que** le couplage du circuit de réglage avec l'étage d'attaque (16) s'effectue en parallèle par l'intermédiaire d'un coupleur optique (30, 22) et d'un transformateur (26), de préférence un transformateur d'impulsions, et les signaux de sortie du coupleur optique et du transformateur sont additionnés dans un étage d'addition (20).
